# EUROPEAN PATENT SPECIFICATION

(11) **EP 3 996 708 B1**
(45) Date of publication and mention of the grant of the patent: **22.04.2026**
(21) Application number: 19742324.7
(22) Date of filing: 12.07.2019
(51) Int. Cl.: A61K 31/4162, A61P 35/00

(54) **PHARMACEUTICAL COMPOSITIONS COMPRISING INHIBITORS OF OPA1 FOR USE IN THE TREATMENT OF CANCER**
PHARMACEUTISCHE ZUSAMMENSETZUNG ENTHALTEND OPA1 INHIBITOR ZUR BEHANDLUNG VON KREBS
COMPOSITION PHARMACEUTIQUE COMPRENANT DES INHIBITEURS DU OPA1 POUR TRAITER LE CANCER

(43) Date of publication of application: 18.05.2022
(73) Proprietor: Università Degli Studi Di Padova, 35122 Padova (IT); Fondazione per la Ricerca Biomedica Avanzata Onlus - V.I.M.M., 35129 Padova (IT)
(72) Inventor: SCORRANO, Luca, 35122 Padova (IT); QUIRIN, Franziska Charlotte, 35122 Padova (IT); PELLATTIERO, Anna, Padova 35122 (IT)
(74) Representative: Leone, Mario
(86) International application number: PCT/EP2019/068846
(87) International publication number: WO 2021/008669

(56) References cited:
- WO-A2-2010/091878
- OMORI AKIKO ET AL: "Exploring the role of mitochondrial dynamics on prostatic development and cancer", BIOCHIMICA ET BIOPHYSICA ACTA. BIOENERGETICS, vol. 1857, 2016, XP029568543, ISSN: 0005-2728, DOI: 10.1016/J.BBABIO.2016.04.240
- PELLATTIERO ANNA ET AL: "Development of a New Chemical Inhibitor of OPA1 Activity to Increase Cancer Cell Sensitivity Toward Apoptosis", BIOCHIMICA ET BIOPHYSICA ACTA. BIOENERGETICS, vol. 1859, September 2018 (2018-09-01), XP085543111, ISSN: 0005-2728, DOI: 10.1016/J.BBABIO.2018.09.185
- SEHRAWAT ANURADHA ET AL: "Withaferin A-mediated apoptosis in breast cancer cells is associated with alterations in mitochondrial dynamics", MITOCHONDRION, vol. 47, July 2019 (2019-07-01), pages 282 - 293, XP085722860, ISSN: 1567-7249, DOI: 10.1016/J.MITO.2019.01.003

## Description

### Background of the invention

The present invention refers to the pharmaceutical field and more in particular to small molecules inhibiting the GTPase activity of the mitochondrial dynamin related protein OPA1, and enhancing the release of cytochrome c from mitochondria, in response to a pro-apoptotic stimulus, thus sensitizing cells to the induction of apoptotic cell death. Said molecules can be used for the treatment of cancer and cancer-induced angiogenesis involving the upregulation of OPA1 levels and function.

### State of the art

Mitochondria are multifunctional organelles that regulate several fundamental physio-pathological cellular processes. They play crucial roles in metabolism, bioenergetics (Danial, N.N., Gramm, C.F., Scorrano, L., Zhang, C.Y., Krauss, S., Ranger, A.M., Datta, S.R., Greenberg, M.E., Licklider, L.J., Lowell, B.B., et al. (2003) BAD and glucokinase reside in a mitochondrial complex that integrates glycolysis and apoptosis, Nature 424, 952-956.), Ca²⁺ signaling (Rizzuto, R., Bernardi, P., and Pozzan, T. (2000, Mitochondria as all-round players of the calcium game, J Physiol 529 Pt 1, 37-47), oxidative stress response (Orrenius, S., Gogvadze, V., and Zhivotovsky, B. (2007), Mitochondrial oxidative stress: implications for cell death, Annu Rev Pharmacol Toxicol 47, 143-183), and integration and amplification of apoptotic signals (Green, D.R., and Kroemer, G. (2004), The pathophysiology of mitochondrial cell death, Science 305, 626-629). Mitochondrial morphology is the result of a fine balance between fission and fusion processes, mediated by the "mitochondrial-shaping" proteins. The dynamin related protein (Drp) 1, along with its receptor proteins mitochondrial fission factor (Mff) and fission (Fis) 1, mediate mitochondrial fission, while fusion is controlled by mitofusins (Mfn) 1 and 2 at the outer membrane, and by optic atrophy (Opa) 1 at the inner membrane. Mitochondrial dynamins ensure organelle plasticity, but are also associated with several human disorders and tumors (Wallace, D.C. (2005), A mitochondrial paradigm of metabolic and degenerative diseases, aging, and cancer: a dawn for evolutionary medicine. Annu Rev Genet 39, 359-407). Mitochondria-shaping proteins were first linked to cancer because of their role in apoptosis, whose inhibition is a hallmark of cancer cells (Hanahan, D., and Weinberg, R.A. (2011), Hallmarks of cancer: the next generation. Cell 144, 646-674). Subsequent studies revealed that changes in mitochondrial fission-fusion balance confer cancer cells the capacity to adapt their metabolism to environmental modifications, such as hypoxia, starvation, stress signals and even drug treatments (Patten, D.A., Wong, J., Khacho, M., Soubannier, V., Mailloux, R.J., Pilon-Larose, K., MacLaurin, J.G., Park, D.S., McBride, H.M., Trinkle-Mulcahy, L., et al. (2014), OPA1-dependent cristae modulation is essential for cellular adaptation to metabolic demand, EMBO J 33, 2676-2691; Rossignol, R., Gilkerson, R., Aggeler, R., Yamagata, K., Remington, S.J., and Capaldi, R.A. (2004), Energy substrate modulates mitochondrial structure and oxidative capacity in cancer cells, Cancer Res 64, 985-993). Apoptosis is a natural barrier to uncontrolled cell proliferation and, thus, a crucial cancer defense. Indeed, dysregulation of apoptotic pathway is an hallmark of cancer. Tumor cells evolved different strategies to limit or overcome apoptosis, for example loss of tumor suppressors, as p53, activation of proto-oncogenes, upregulation of antiapoptotic protein, like Bcl-2 or IAPs, or downregulation of proapoptotic factors, as Bax and Bak. The recent advances in high-throughput technologies allowed the genetic characterization of several tumor types and expanded the number of molecular targets. These advances rapidly increased the rush to the personalized cancer therapies. However, many challenges need to be overcome, as tumor heterogeneity and evolution, potential morbidity of biopsies, but especially the lack of effective drugs against most genomic aberrations and the absence of a specific genetic profile for each tumor type (Meric-Bernstam, F., and Mills, B.G. (2012). Overcoming implementation challenges of personalized cancer therapy. Nature Reviews Clinical Oncology 9, 542). On the other hand, conventional cancer drugs, as etoposide, cisplatin or doxorubicin, still widely employed in the clinics, exert their function by indirectly inducing apoptosis by engaging signaling pathways that lie upstream of mitochondria (Fulda, S., Galluzzi, L., and Kroemer, G. (2010). Targeting mitochondria for cancer therapy, Nature Reviews Drug Discovery 9, 447-464). Unfortunately, cancer cells often become resistant to these treatments through modifications of the apoptotic mechanisms. For these reasons, drugs that directly target mitochondria are becoming of great interest because they reactivate cell death by bypassing the resistance mechanisms and sensitizing chemoresistant cells. Among all mitochondria-shaping proteins, OPA1 plays a central antiapoptotic role. Upon apoptosis induction, Bcl-2 family proteins permeabilize OMM and mitochondria undergo an ultrastructural remodeling (Scorrano, L. (2009). Opening the doors to cytochrome c: changes in mitochondrial shape and apoptosis, Int J Biochem Cell Biol 41, 1875-1883; Scorrano, L., Ashiya, M., Buttle, K., Weiler, S., Oakes, S.A., Mannella, C.A., and Korsmeyer, S.J. (2002), A distinct pathway remodels mitochondrial cristae and mobilizes cytochrome c during apoptosis; Dev Cell 2, 55-67; Scorrano, L., and Korsmeyer, S.J. (2003). Mechanisms of cytochrome c release by proapoptotic BCL-2 family members. Biochem Biophys Res Commun 304, 437-444; Scorrano, L., Oakes, S.A., Opferman, J.T., Cheng, E.H., Sorcinelli, M.D., Pozzan, T., and Korsmeyer, S.J. (2003), BAX and BAK regulation of endoplasmic reticulum Ca2+: a control point for apoptosis, Science 300, 135-139). OPA1 controls this process, independently from its role in mitochondrial fusion. Indeed, OPA1 oligomers, containing both the membrane bound and the soluble forms of the protein, participate in the formation and in the maintenance of tight cristae, sequestrating cytochrome c in the intra-cristae compartment. During apoptosis, OPA1 oligomers are early targets of BID, BIM-1, BNIP3 (Frezza, C., Cipolat, S., Martins de Brito, O., Micaroni, M., Beznoussenko, G.V., Rudka, T.,Bartoli, D., Polishuck, R.S., Danial, N.N., De Strooper, B., et al. (2006b), OPA1 Controls Apoptotic Cristae Remodeling Independently from Mitochondrial Fusion. Cell 126, 177-189; Landes, T., Emorine, L.J., Courilleau, D., Rojo, M., Belenguer, P., and Arnaune-Pelloquin, L. (2010), The BH3-only Bnip3 binds to the dynamin Opa1 to promote mitochondrial fragmentation and apoptosis by distinct mechanisms. EMBO Rep 11, 459-465; Yamaguchi, R., Lartigue, L., Perkins, G., Scott, R.T., Dixit, A., Kushnareva, Y., Kuwana, T., Ellisman, M.H., and Newmeyer, D.D. (2008), Opa1-mediated cristae opening is Bax/Bak and BH3 dependent, required for apoptosis, and independent of Bak oligomerization. Mol Cell 31, 557-569), and they are rapidly dissociated leading to cristae widening, cytochrome c mobilization from cristae for its subsequent release into the cytosol. Downregulation of OPA1 leads to mitochondrial network fragmentation and increases cell sensitivity towards apoptosis, while mutations that abolish OPA1 catalytic activity impair its antiapoptotic function. Interestingly, OPA1 is overexpressed in different types of cancers where it correlates with an increased chemotherapy resistance and a lower survival rate. For example, OPA1 is overexpressed in lung adenocarcinoma (LADC) patients where it increases cisplatin resistance via inactivation of caspase-dependent apoptosis; knockdown of OPA1 sensitizes hepatocellular carcinoma cells to cytotoxic treatment and Akt-dependent downregulation of OPA1 facilitates cytochrome c release and apoptosis in lung cancer cells (Fang, H.Y., Chen, C.Y., Chiou, S.H., Wang, Y.T., Lin, T.Y., Chang, H.W., Chiang, I.P., Lan,K.J., and Chow, K.C. (2012), Overexpression of optic atrophy 1 protein increases cisplatin resistance via inactivation of caspase-dependent apoptosis in lung adenocarcinoma cells, Hum Pathol 43, 105-114.; Kong, B., Wang, Q., Fung, E., Xue, K., and Tsang, B.K. (2014), p53 is required for cisplatin induced processing of the mitochondrial fusion protein L-Opa1 that is mediated by the mitochondrial metallopeptidase Oma1 in gynecologic cancers. J Biol Chem 289, 27134-27145; Zhao, X., Tian, C., Puszyk, W.M., Ogunwobi, O.O., Cao, M., Wang, T., Cabrera, R., Nelson, D.R., and Liu, C. (2013), OPA1 downregulation is involved in sorafenib-induced apoptosis in hepatocellular carcinoma. Lab Invest 93, 8-19). OPA1 was identified as a member of a group of eight prognosis-related genes, including EBAG9, MTDH, ATP6V1C1, ATCL6A, BCL6, SENP5 and KRAS, likely to be important cross-cancer target genes for therapies and perhaps also associated with overall patient's survival rate, due to their recurrent copy number amplification across pan-cancer (Wee, Y., Liu, Y., Lu, J., Li, X., and Zhao, M. (2018), Identification of novel prognosis-related genes associated with cancer using integrative network analysis, Sci Rep 8, 3233). In addition, not only genes controlling mitochondrial dynamins were found to be recurrently amplified in multiple tumor types, but deletion of OPA1 (and DRP1) in a proof of principle experiment increased sensitivity of multiple cancer cells , such as lung and ovarian, toward specific SMAC-mimetic anticancer drugs (Anderson, G.R., Wardell, S.E., Cakir, M., Yip, C., Ahn, Y.R., Ali, M., Yllanes, A.P., Chao, C.A., McDonnell, D.P., and Wood, K.C., 2018, Dysregulation of mitochondrial dynamins proteins are a targetable feature of human tumors. Nat Commun 9, 1677).

Closest prior art is international patent application, publication n. WO2010091878 disclosing that cancer is a mitochondrial disorder correlated to mithocondrial dysfunctions.

### Technical problem

There is a strong need for specific inhibitors of mitochondrial proteins belonging to the superfamily of dymamins and in particular OPA1, since it is up-regulated in different types of cancer, and it is involved in resistance to chemotherapy and to target anti-tumor therapy.

In view of the findings of the prior art, the same inventors investigated the possibility that chemical inhibition of OPA1 GTPase activity could mimic the crucial mutations in the GTPase domain, being involved in apoptosis. Therefore, they setup and implemented an in vitro high-throughput screening assay to test a library of chemical compounds for their effect on the GTPase activity of recombinant, purified OPA1.

### Object of the invention

The technical problem is solved by providing a pharmaceutical composition comprising a therapeutically effective amount of at least one compound of formula (I): 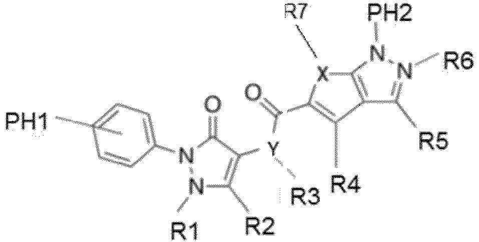 wherein
X and Y, the same or different, is N,O,S,B,P
PH1 and PH2, the same or different, is phenyl,
optionally substituted with H, OH, halogen, linear or branched saturated or unsaturated C1-C20 alkyl, optionally substituted by one or more amino, imino, hydroxyl and aldehyde groups, and/or can contain one or more nitrogen groups, optionally substituted by a C1-C6 linear or branched, saturated or unsaturated alkyl group;
R9C=CR13, R9C≡CR13, R9(Halogen), R9OH, R9COR13, R9CHO, R9CO(halogen), R9OCOOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9)(OR13) (OR14) (OR15), R9(CO)O(CO)R14, CO(R9)₂, , CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO-(CH₂)ₙ-COOH, CH=NO-(CH₂) ₙ-NH₂, CH=NO- (CH₂)ₙ-OH, (CH₂)ₙNHCO-R9, (CH₂)ₙ-R9, CH=NO-R9, CH₂OH, (CH₂)ₙNH-R9, (CH₂)ₙN(R9)₂, (CH₂)ₙ-R10, C(=NH)NH₂, C=CCOOH CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS,
R1, R2, R3, R4,R5, R6, R7, the same or different, is H, OH, halogen, optionally substituted by one or more amino, imino, hydroxyl and aldehyde groups, and/or can contain one or more nitrogen groups, optionally substituted by a C1-C6 linear or branched, saturated or unsaturated alkyl group, CHO, NH₂, R9C=CR13, R9C≡CR13, R9(Halogen), R9OH, R9COR13, R9CHO, R9CO(halogen), R90COOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9) (OR13) (OR14) (OR15), R9(CO)O(CO)R14, CO(R9)₂, CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO- (CH₂)ₙ-COOH, CH=NO-(CH₂)ₙ-NH₂, CH=NO-(CH₂)ₙ-OH, (CH₂)ₙNHCO-R9, (CH₂)ₙ-R9, CH=NO-R9, CH₂OH, (CH₂)ₙNH-R9, (CH₂)ₙN(R9)₂, (CH₂)ₙ-R10, C(=NH)NH₂, C≡CCOOH CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS, phenyl, aryl
R8 is H, OH, COOH, NH₂,
R9,R13,R14 is, the same or different, H, linear or branched saturated or unsaturated C₁-C₂₀ alkyl
n is an integer n is an integer from 1 to 20
R10 is OH, NH₂, N-R11R12,
R11 and R12, the same or different is H, C₁-C₁₀ alkyl linear or branched saturated or unsaturated,
their enantiomeric and/or diastereomeric mixtures, their pharmaceutically acceptable salts, their solvates, hydrates;
and pharmaceutically acceptable vehicles or excipients for use in the treatment of cancer.

### Brief Description of the Drawings

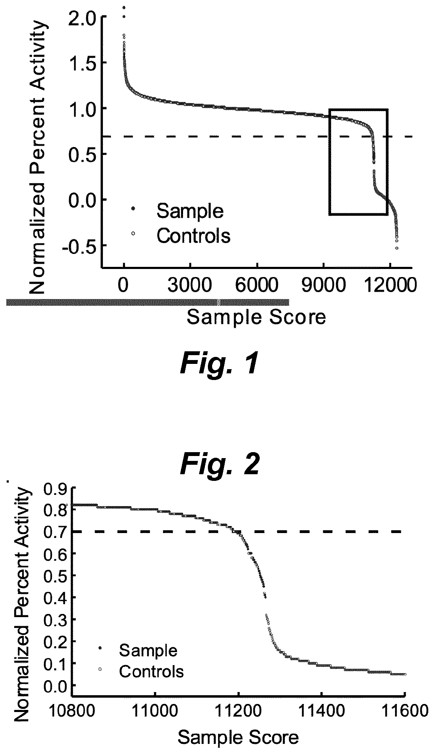

Figure 1 shows in graph the normalized percent inhibition (NPI) values of each compound and control samples plotted against their z-score. The dashed line indicates the threshold set at 0.7 corresponding to 30% inhibition of the measured GTPase activity of OPA1. Data represent the average of two HTS replicates.
Figure 2 shows the magnification of a plot reporting compounds with NPI between 0 and 1, wherein compounds with NPI < 0.69 were selected for the subsequent analysis and in graph the results of in silico docking analysis of the interaction of MYLS22with rOPA1.

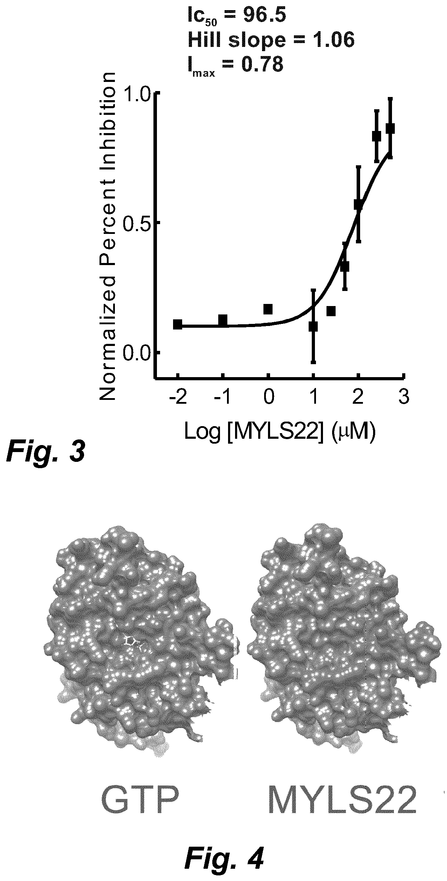

Figure 3 show the dose-response curves for inhibition of rOPA1 GTPase activity by OPA1 inhibitor MYLS22. Normalized percent of inhibition (NPI) values are plotted against the logarithmic concentration of the inhibitor. Data are normalized using EDTA-containing samples as positive controls and vehicle (DMSO) containing samples as negative controls. A four parametric curve was fit to the data to obtain the stated half-maximal inhibition value (IC50), maximum inhibition (I50) and Hill slope values using OriginPro software. Data represent the mean of 3 independent experiments ± SEM.
Figure 4 shows the surface representations of the predicted GTPase domain of rOPA1 in complex with GTP (left) or MYLS22 (right).

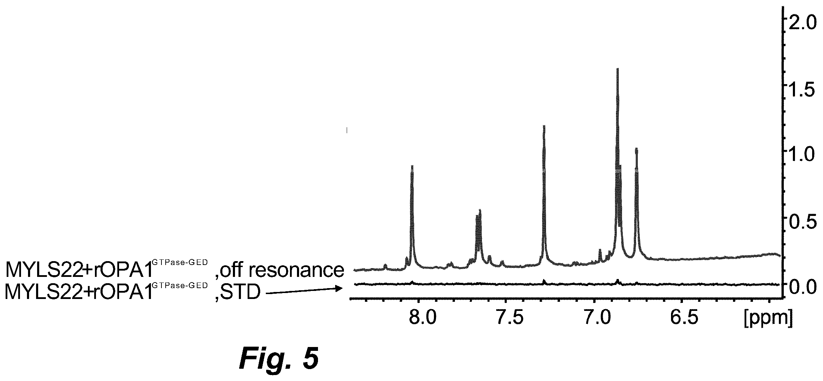

Figure 5 shows the rOPA1-MYLS22 interaction measured using the saturation-transfer difference (STD) NMR technique. The lower NMR spectrum shows the aromatic region of MYLS22, while the above spectrum represents the difference spectrum after selective saturation of OPA1.

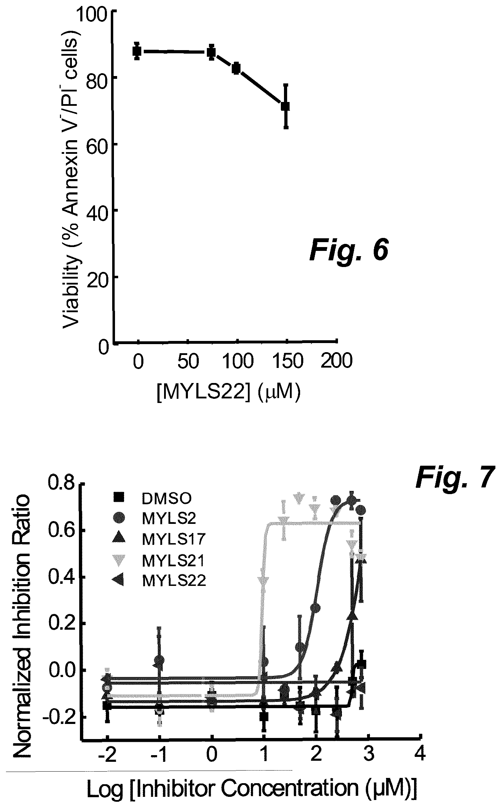

Figure 6 shows results of cellular toxicity of MYLS22. MYLS22 does not induce cell death, WT MEFs were incubated with the indicated concentrations of MYLS22. After 24h, viability was measured cytofluorimetrically as the percentage of Annexin V and propidium iodide negative cells. Data represents average ± SEM of six independent experiments.
Figure 7 shows the specificity of MYLS22 inhibition of OPA1 GTPase activity. Dose response curves of recombinant Dynamin-1 activity after incubation with OPA1 inhibitors. Data were fitted with a four-parametric curve, values represent mean ± SEM of two independent experiments.

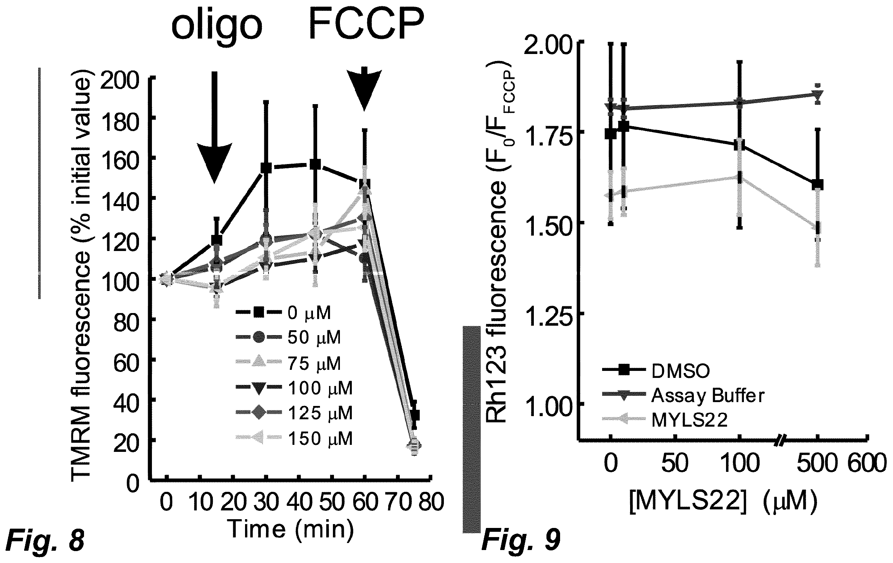

Figure 8 shows results of mitochondrial toxicity of MYLS22. MYLS22 does not induce mitochondrial membrane potential depolarization in WT MEFs. Cells were treated with MYLS22 and TMRM fluorescence was recorded in response to oligomycin and the uncoupler FCCP. Cells treated with MYLS22 do not present a reduction of the membrane potential.
Figure 9 shows results of mitochondrial toxicity of MYLS22. MYLS22 does not induce mitochondrial potential depolarization in isolated mitochondria. Effect of MYLS22 on isolated mitochondrial membrane potential represented by the ratio of rhodamine 123 (Rh123) fluorescence before and after treatment with the uncoupler FCCP. Mitochondria were treated with MYLS22 in the presence of glutamate/malate (Glu/Mal) as complex I substrate.. Data represents mean ± SEM of three independent experiments.

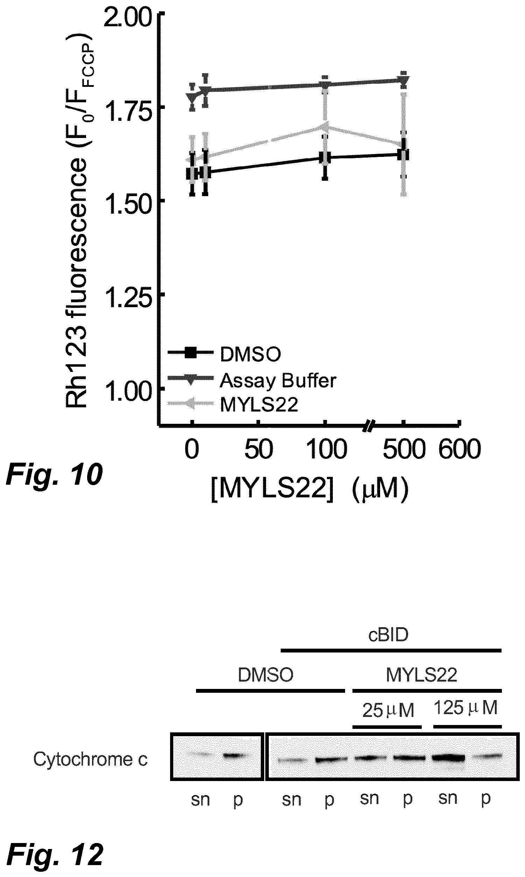

Figure 10 shows results of mitochondrial toxicity of MYLS22. MYLS22 does not induce mitochondrial potential depolarization in isolated mitochondria. Effect of MYLS22 on isolated mitochondrial membrane potential represented by the ratio of rhodamine 123 (Rh123) fluorescence before and after treatment with the uncoupler FCCP. Mitochondria were treated with MYLS22 with succinate as complex II substrate. Data represents mean ± SEM of three independent experiments.

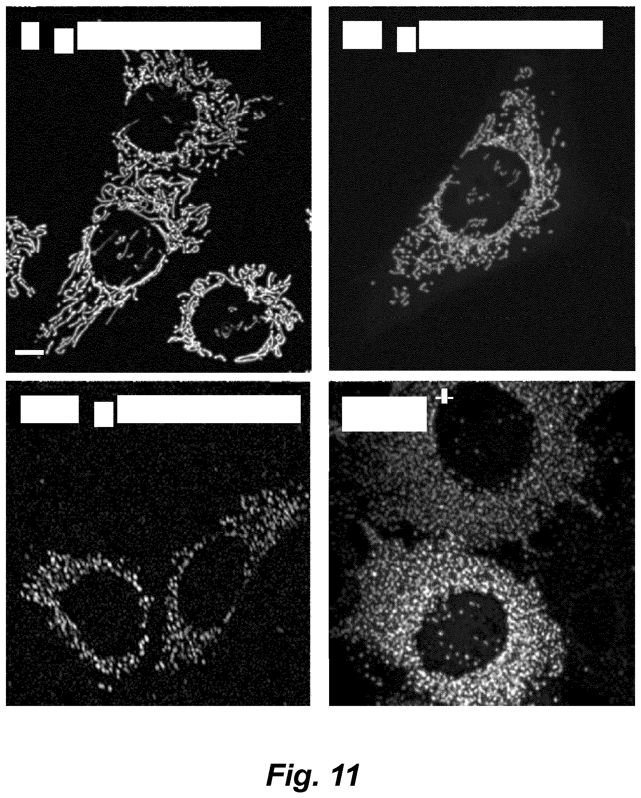

Figure 11 shows representative confocal images of changes in mitochondrial morphology induced by MYLS22. WT MEFs expressing mitochondrially targeted YFP (mtYFP) were treated with 50 µM (upper right) or 100 µM (bottom left) MYLS22 or DMSO (upper left) for 24h. Bottom right: representative image of knockdown of mitochondrial network fragmentation induced by OPA1 knockdown. Bar = 10 µm.
Figure 12 shows immunoblot of in vitro cytochrome c release by recombinant cBID protein. Mitochondria purified by mouse liver were incubated with cBID and the indicated concentrations of MYLS22. Mitochondria were subsequently centrifuged and pellet (p) and supernatant (s) were assayed for cytochrome c release.

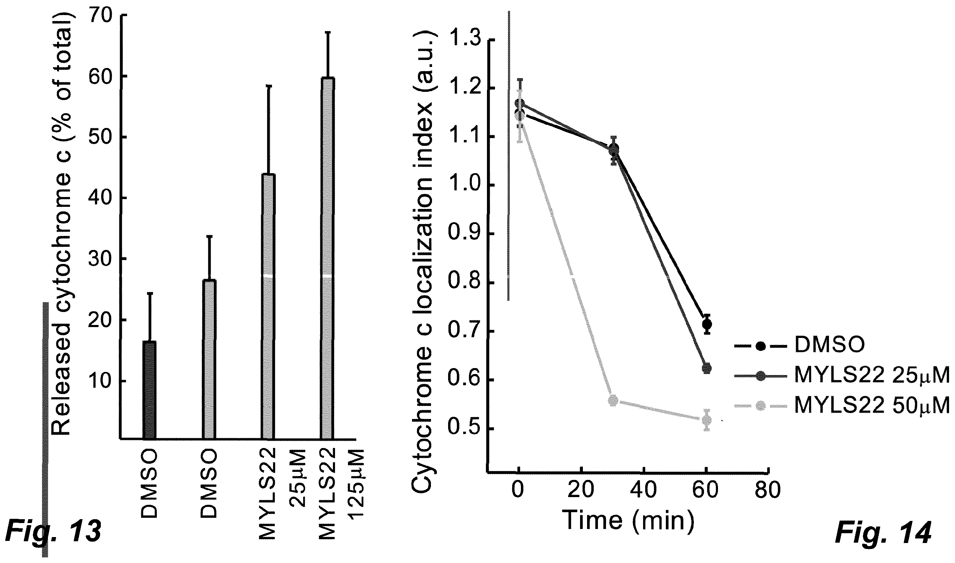

Figure 13 shows in graph cytochrome c release from mitochondrial cristae. Isolated mitochondria from mouse liver were treated as in figure 12 and cytochrome c release was measured by ELISA. Data represent mean ± SEM of three independent experiments.
Figure 14 shows in graph results of localization index for cytochrome c. WT MEFs were treated with 50 µM MYLS22 (bottom) or left untreated (upper). Then cells were treated with 1 mM H2O2 for 30 min or 60 min, fixed and immunostained for cytochrome c and TOM20. Bar corresponds to 10 µm. Localization index was calculated from 30 images randomly selected. Data represent mean ± SEM of three independent experiments.

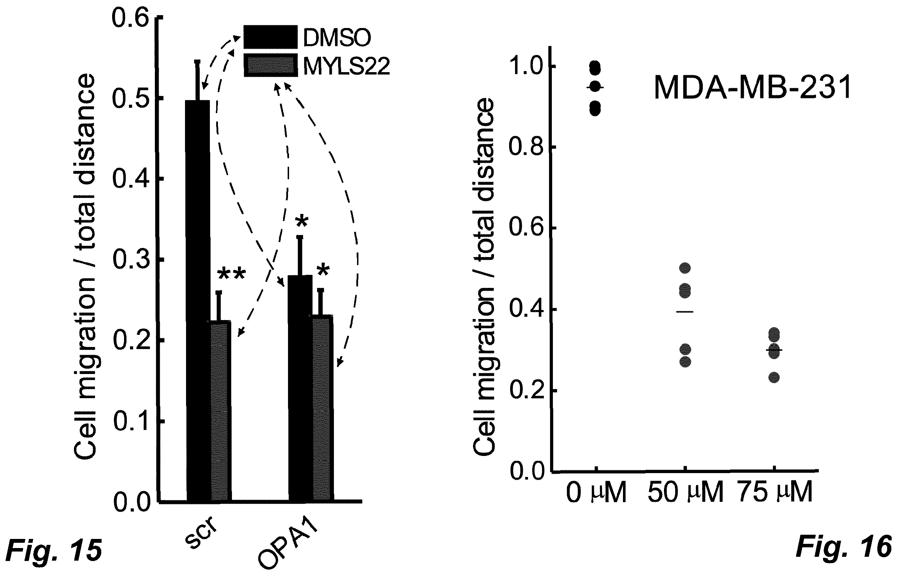

Figure 15 shows in graph effect of MYLS22 on breast cancer cell migration. MDA-MB-231 cells were transfected with scr or OPA1 siRNA oligos and after 72 h, were incubated with 20 µM of MYLS22 or DMSO as negative control. Cells migration was measured by scratch assay. The migration of the cells into the wound was measured after 6 hours. Data represent the mean ± SEM of three independent experiments.
Figure 16 shows results of lung adenocarcinoma PC9 cells migration measured by scratch assay. Cells were incubated with the reported concentration of MYLS22 or DMSO, as negative control, and the migration of the cells into the wound was measured after 8 hours. Data represent the mean ± SEM of five independent experiments.

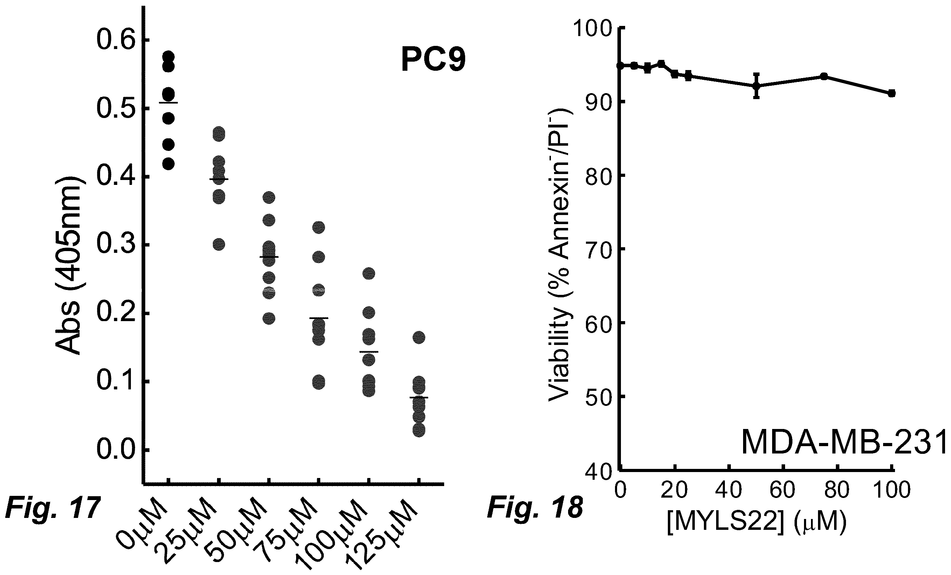

Figure 17 shows results of lung adenocarcinoma PC9 cells proliferation measured by BrdU incorporation. Prior to BrdU measurement, cells were incubated 24h with the indicated concentration of MYLS22 or DMSO, as negative control. Data represent the mean ± SEM of nine independent experiments.
Figure 18 shows the effect of MYLS22 on apoptotic cell death induction. MYLS22 does not induce cell death in MDA-MB-231 cells. The cells were incubated with the indicated concentrations of MYLS22 and after 24h, viability was measured cytofluorimetrically as the percentage of Annexin V and propidium iodide negative cells. Data represents average ± SEM of three independent experiments.

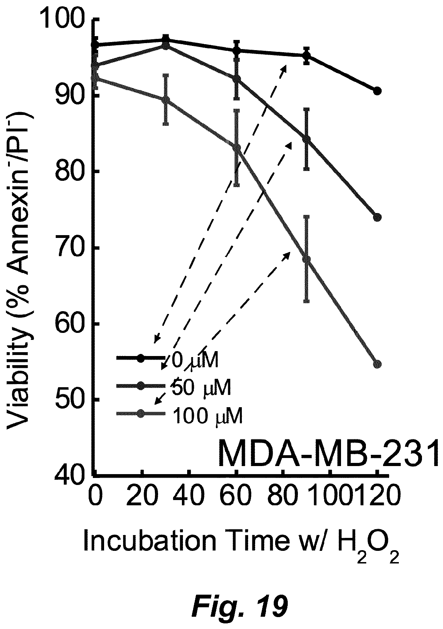

Figure 19 shows that MYLS22 enhances MDA-MB-231 cells death after an apoptotic stimulus. The cells were incubated with 0, 50 or 100 µM MYLS22 and 1 mM H₂O₂. At the indicated timepoints, cells viability was measured cytofluorimetrically as the percentage of Annexin V and propidium iodide negative cells. Data represents average ± SEM of three independent experiments.

### Detailed Description of the Invention

The pharmaceutical composition of the present invention comprises a therapeutically effective amount of at least one compound of formula (I): 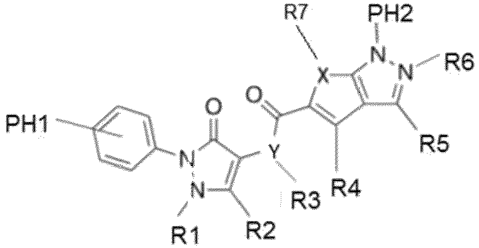 wherein
X and Y, the same or different, is N,O,S,B,P
PH1 and PH2, the same or different, is phenyl,
optionally substituted with H, OH, halogen, linear or branched saturated or unsaturated C₁-C₂₀ alkyl, optionally substituted by one or more amino, imino, hydroxyl and aldehyde groups, and/or can contain one or more nitrogen groups, optionally substituted by a C₁-C₂₀ linear or branched, saturated or unsaturated alkyl group;
R9C=CR13, R9C≡CR13, R9(Halogen), R9OH, R9COR13, R9CHO, R9CO(halogen), R9OCOOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9)(OR13) (OR14) (OR15), R9(CO)O(CO)R14, CO(R9)₂, , CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO-(CH₂)ₙ-COOH, CH=NO- (CH₂) ₙ-NH₂, CH=NO-(CH₂)ₙ-OH, (CH₂)ₙNHCO-R9, (CH₂)ₙ-R9, CH=NO-R9, CH₂OH, (CH₂)ₙNH-R9, (CH₂)ₙN(R9)₂, (CH₂)ₙ-R10, C(=NH)NH₂, C≡CCOOH CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS,
R1, R2, R3, R4,R5, R6, R7, the same or different, is H, OH, halogen, optionally substituted by one or more amino, imino, hydroxyl and aldehyde groups, and/or can contain one or more nitrogen groups, optionally substituted by a C₁-C₂₀ linear or branched, saturated or unsaturated alkyl group, CHO, NH₂, R9C=CR13, R9C≡CR13, R9(Halogen), R9OH, R9COR13, R9CHO, R9CO(halogen), R9OCOOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9) (OR13) (OR14) (OR15), R9(CO)O(CO)R14, CO(R9)₂, , CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO- (CH₂)ₙ-COOH, CH=NO- (CH₂)ₙ-NH₂, CH=NO- (CH₂)ₙ-OH, (CH₂)ₙNHCO-R9, (CH₂)ₙ-R9, CH=NO-R9, CH₂OH, (CH₂)ₙNH-R9, (CH₂)ₙN(R9)₂, (CH₂)ₙ₋R10, C(=NH)NH₂, C≡CCOOH CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS, phenyl, aryl, bicyclic, heterocyclic,
R8 is H, OH, COOH, NH₂,
R9,R13,R14 is, the same or different, H, linear or branched saturated or unsaturated C₁-C₂₀ alkyl
n is an integer n is an integer from 1 to 20
R10 is OH, NH₂, N-R11R12,
R11 and R12, the same or different is H, C₁-C₁₀ alkyl linear or branched saturated or unsaturated,
their enantiomeric and/or diastereomeric mixtures, their pharmaceutically acceptable salts, their solvates, hydrates

The composition for use in the treatment of cancer.

Where the compounds of formula (I) can exhibit tautomerism, the formula is intended to cover all tautomers; the invention includes within its scope all the possible stereoisomers, Z and E isomers, optical isomers, enantiomers and their mixtures.

Also the pharmaceutically acceptable salts are included in the scope of the invention. Pharmaceutically acceptable salts are salts which retain the biological activity of the base compound and are derived from such known pharmacologically acceptable acids such as, e. g., hydrochloric, hydrobromic, sulfuric, phosphoric, nitric, fumaric, succinic, oxalic, malic, tartaric, maleic, citric, methanesulfonic or benzoic acid and others commonly used in the art, see for example Pharmaceutical Salts and Co-crystals; Editors: Johan Wouters, Luc Quéré, RSC Publishing.

Some compounds of formula (I) can also be prodrugs of the active forms.

The treatment of cancer is exerted by the compounds of the present invention since they act as inhibitors of GTPase activity of OPA1, as enhancers of the release of cytochrome C from mitochondria and as apopotosis inducers.

Preferably in the compound of formula (I) PH1 and PH2 are phenyl, R1 R2 and R5 are CH₃, R6 R7 are H, X is S, Y is N. The most preferred compound is N-(1,5-dimethyl-3-oxo-2-phenyl-2,3-dihydro-1H-pyrazol-4-YL) 3-methyl-1-phenyl-1H-thieno[2,3-c]pyrazole-5-carboxamide (C24H21N5O2S) which is named MYLS22 in the examples.

Optionally the pharmaceutical composition further comprises a chemotherapeutic drug.

Preferably chemotherapeutic drug is selected from the group consisting of doxorubicin, etoposide and cisplatin. Preferably cancer is breast cancer, lung cancer.

The pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilizing agents, dispersing agents, suspension agents, and emulsifying agents.

Preferably in the compound of formula (I) PH1 and PH2 are phenyl, R1 R2 and R5 are CH₃, R6 R7 are H, X is S, Y is N. The most preferred compound is N-(1,5-dimethyl-3-oxo-2-phenyl-2,3-dihydro-1H-pyrazol-4-YL) 3-methy1-1-phenyl-1H-thieno[2,3-c]pyrazole-5-carboxamide (C24H21N5O2S) which is named MYLS22 in the examples.

Preferably cancer is breast cancer, lung cancer.

### Examples

### Materials and methods

Cell culture: WT OPA1 SV40 transformed mouse adult fibroblast (MAFs) were generated from WT mice as previously described (Cogliati, S., Frezza, C., Soriano, M.E., Varanita, T., Quintana-Cabrera, R., Corrado, M., Cipolat, S., Costa, V., Casarin, A., Gomes, L.C., et al. (2013). Mitochondrial cristae shape determines respiratory chain supercomplexes assembly and respiratory efficiency. Cell 155, 160-171). Cell lines were cultured in Dulbecco's Modified Eagle Medium (DMEM, Invitrogen) supplemented with 10% FBS (Invitrogen), 2 mM Glutamine, 50 U/ml Penicillin, 50 µg/ml Streptomycin, 50 µg/ml Uridine, and 0.1 mM non-essential aminoacids (Invitrogen). Production and purification of recombinant proteins: OPA1 mouse transcript variant 2 (NM_133752) was amplified from position 502 to the stop codon to produce recombinant OPA1 (rOPA1) lacking the N-terminal mitochondrial targeting sequence and the transmembrane domain. The PCR product was cloned into pET21+ (Novagen) which adds a His-tag to the C-terminus of the encoded protein and expressed in E. coli. Protein expression was induced with 0.5 µM IPTG for 20 hours at 18 °C. Cells were collected by centrifugation and the pellet was resuspended in lysis buffer (40 mM Hepes/KOH, 500 mM NaCl, 10% glycerol, 5 mM MgCl₂, 5 mM β-mercaptoethanol, 0.5% Triton-X-100, 2% Tween-20, 1 mM PMSF, 20 mM Imidazole, Roche protease inhibitor cocktail, pH 8.0), were lysed by sonication and cell debris was removed by centrifugation at 14000g for 45 minutes, 4 °C. rOPA1 was purified by Ni-NTA batch chromatography. Prior to elution, beads were washed with 1 mM ATP and 10 mM MgCl2 in lysis buffer for 30 minutes at room temperature followed by a washing step in wash buffer (40 mM Hepes/KOH, 300 mM NaCl, 0.05% Triton-X-100, 5 mM MgCl2, 5 mM β-mercaptoethanol, 10% glycerol, 20 mM Imidazole, pH 8.0). rOPA1 was eluted with increasing concentrations of imidazole in elution buffer (40 mM Hepes/KOH, 0.05% Triton-X-100, 5 mM β-mercaptoethanol, 10% glycerol, pH 7.4). Imidazole was removed by dialysis and the protein was concentrated in storage buffer (40 mM Hepes/KOH, 0.05% Triton-X-100, 0.3 mM TCEP, 10% glycerol) and stored at -80 °C until use. p7/p15 recombinant cBID was expressed, purified and cleaved with caspase-8 as described in (Frezza, C., Cipolat, S., Martins, d.B., Micaroni, M., Beznoussenko, G.V., Rudka, T., Bartoli, D., Polishuck, R.S., Danial, N.N., De Strooper, B., et al. (2006). OPA1 Controls Apoptotic Cristae Remodeling Independently from Mitochondrial Fusion. Cell 126, 177-189).

Malachite Green GTPase activity assay: GTPase activity was measured using a modified version of the previously described Malachite Green assay (Leonard, M., Doo Song, B., Ramachandran, R., and Schmid, S.L. (2005). Robust Colorimetric Assays for Dynamin's Basal and Stimulated GTPase Activities. In Methods in Enzymology (Academic Press), pp. 490-503), based on the change of absorbance of malachite green in the presence of free PO4 ions that results from the hydrolysis of GTP into GDP. A mixture containing containing 8 mM ammonium molybdate and 1.5 mM malachite green was freshly prepared the day of use. The change in absorbance molybdate and the free phosphate form a phosphomolybdate complex (Cogan E. B., Birrell G. B., Griffith O. H. (1999). A robotics-based automated assay for inorganic and organic phosphates. Anal Biochem. 271, 29-35) that then binds the malachite green dye resulting in a change of absorbance depending on the contained phosphate concentration that was measured at 595 nm after a 15 minutes incubation period at room temperature. The assay was performed in 96 well-plates in a final volume of 190 µl/well. To start the hydrolysis reaction, 10 µl of 0.5 µM of rOPA1 in 40 mM Hepes/KOH pH 7.4 was mixed with 10 µl of 500 µM GTP and 10 mM MgCl2 and incubated for 2 hours at 37 °C. The reaction was then halted by adding to each well 5 µl of 500 µM EDTA, which chelates the Mg2+ required for GTP hydrolysis. To measure the phosphate content, 150 µL of the Malachite Green-ammonium molybdate solution (1 mM Malachite Green, 10 mM ammonium molybdate in 1N HCl) was added at each well. After 15 minutes incubation at 25 °C, was measured the absorbance at 595 nm using a microplate reader. Phosphate concentration of each sample was calculated from a standard curve ranging from 1 to 100 µM Pi generated for each experiment. To test the inhibition of rOPA1 GTPase activity by the inhibitor, 5 µl of the inhibitor dissolved in DMSO, or DMSO alone as solvent control, were incubated with rOPA1 for 20 minutes at 37 °C before to initiating the hydrolysis reaction. As positive control of OPA1 activity inhibition, 5 µl of 500 µM EDTA were added before incubation with GTP to prevent the hydrolysis reaction. Samples with only buffer and 500 µM GTP were used as blank controls.

Analysis of protein expression: Protein concentration from total cell extracts or mitochondria lysates was assessed with Bradford reagent (Bio-Rad) and equal amounts of proteins were separated by 3-8% Tris-Acetate SDS-PAGE (NuPage, Invitrogen). Fractions of the affinity purification of rOPA1 were resolved by 4-12% Bis-Tris SDS-PAGE (NuPage, Invitrogen). Western blotting was performed by transferring proteins onto PVDF membranes (Merck) and probed using anti-OPA1 monoclonal (1:1000, BD PharmingenTM), anti-cytochrome c monoclonal (1:1000, BD PharmingenTM) anti-actin (1:15000, Merk) antibodies. Isotype matched secondary antibodies were conjugated to horseradish peroxidase and chemiluminescence (Amersham) was detected using Image Quant LAS 4000 (GE Healthcare Life Sciences).

Molecular modelling and docking analysis: Recombinant OPA1 structure model was built with Phyre2 using an intensive model generation approach, that created a complete full-length model of the protein through a combination of multiple template modeling and simplified ab initio folding simulation, as described in (Kelley, L.A., Mezulis, S., Yates, C.M., Wass, M.N., and Sternberg, M.J. (2015). The Phyre2 web portal for protein modeling, prediction and analysis. Nat Protoc 10, 845-858). The top four protein templates selected to model rOPA1 structure based on heuristics were: (i) gtpase hflx (PDB: c5ady6), (ii) dynamin-1 (PDB: c3zvrA), (iii) dynamin 3 (PDB: c5a3fD), dynamin-1 (PDB: c3snhA). The 93% of the final rOPA1 model was modelled at >90% confidence, 7% (residues: 1-42, 86-91) was modelled ab initio. GTP-binding site was used to dock MYLS22 using the Induced Fit Docking of GLIDE (Glide, version 7.1, Schrödinger, LLC). Pymol (The PyMOL Molecular Graphics System, version 2.2.2; Schrödinger, LLC: New York, 2018) was used for preparing images.

Analysis of cell death: 1 × 10⁵ MEFs were grown in 12-well plates. After 24 h cells were treated with MYLS22 or DMSO, as solvent control. After different timepoints cells were stained with Annexin-V-FICT and propidium iodide (PI), according to manufacturer's protocol (eBioscienceTM). Cell death was measured by flow cytometry (FACSCalibur) as the percentage of Annexin-V and PI positive events.

Mitochondrial isolation and in vitro assays: mitochondria from mouse liver were isolated as described elsewhere (Frezza, C., Cipolat, S., and Scorrano, L. (2007b). Organelle isolation: functional mitochondria from mouse liver, muscle and cultured fibroblasts. Nat Protoc 2, 287-295). To measure cytochrome c release, mitochondria (1 mg/ml final concentration) were treated with 50 pmol/mg cBID in Experimental Buffer (EB; 150 mM KCl, 10 mM Tris MOPS, 10 µM EGTA-Tris, 1 mM Pi and 5 mM glutamate/2.5 mM malate as respiration substrate, pH 7.4) at 25 °C. After 15 min, mitochondria were pelleted by centrifugation at 12000 × g at 4 °C for 10 min and resuspended in the same volume of EB. Quantitative determination of cytochrome c in the pellet and the supernatant fractions was determined using the rat/mouse specific ELISA Cytochrome c Immunoassay (R&D Systems). Respiratory chain dysfunctions and membrane potential were assayed by monitoring the release of rhodamine123 from pre-stained mitochondria. 0.5 mg/ml mitochondria were incubated with 0.3 µM rhodamine 123 (Molecular Probes) in EB and the indicated substrates. Rhodamine 123 fluorescence, before and after the addition of the uncoupler agent FCCP, were measured with a fluorescence spectrometer (Infinite 200 Pro, TECAN) at 25 °C, setting at 485 nm and 538 nm the excitation and the emission wavelengths, respectively.

Live imaging of mitochondrial network: To visualize mitochondrial network, 5 x 10⁴ MEF cells expressing mtYFP were seeded onto 25 mm coverslips and treated with the indicated concentrations of the inhibitor MYLS22. After 24 h, the coverslips were incubated in Hank's Balanced Salt Solution (HBSS) supplemented with 10 mM Hepes and placed on the stage of the microscope. Fluorescence signals were analyzed using IMIC Andromeda system (Fondis Electronic) equipped with ORCA-03G Camera (Hamamatsu), a 60x oil objective (UPLAN × 60 oil, 1.35NA, Olympus). A laser was used to excite at 488 nm and FF01-446/523/600/677 (Semrock) as emission filter. Stacks of 25 images separated by 0.3 µm along the z axis were acquired. Convolution and z-stack project were performed using a plug-in of the ImageJ software (National Institutes of Health). Mitochondrial length was quantified with Image J by measuring the average mitochondrial major axis length of 10 mitochondria per cell in 20 cells per sample.

Mitochondrial membrane potential: For imaging of mitochondrial membrane potential, MEFs grown on coverslips and treated with the MYLS22 inhibitor were loaded with 10 nM TMRM (Molecular Probes) in the presence of 2 µg/ml cyclosporine H, a P-glycoprotein inhibitor, and incubated at 37 °C 30 min. Sequential images of TMRM fluorescence were acquired using a High Content Imaging System Operetta confocal microscope (Perkin Elmer) equipped with Harmony software for images analysis.

Cytochrome c immunolocalization: for cytochrome c immunolocalization, MEFs were grown on coverslips and treated with the indicated concentration of MYLS22 or DMSO. After 24 h cells were treated with 1 mM H2O2 to induce apoptosis. After the indicated times, MEFs were fixed and immunostained, as described in Scorrano, L., and Korsmeyer, S.J. (2003). Mechanisms of cytochrome c release by proapoptotic BCL-2 family members. Biochem Biophys Res Commun 304, 437-444, with Alexa Fluor 488-conjugated anti-cytochrome c antibody and Alexa Fluor 568-conjugated anti-TOM20 antibody. For cytochrome c and TOM20 detection, green and red channel images respectively were acquired simultaneously using two separate color channels on the detector assembly of a Zeiss LSM 700 confocal microscope. The localization index was calculated as described in (Frezza, C., Cipolat, S., and Scorrano, L. (2007a). Measuring mitochondrial shape changes and their consequences on mitochondrial involvement during apoptosis. Methods Mol Biol 372, 405-420 and Petronilli, V., Penzo, D., Scorrano, L., Bernardi, P., and Di Lisa, F. (2001). The mitochondrial permeability transition, release of cytochrome c and cell death. Correlation with the duration of pore openings in situ. J Biol Chem 276, 12030-12034.

Scratch wound migration assay: HUVEC and MDA cells were cultured in 48-well plates in 800 µl of EGM2 for 48 hours and were wounded with the head of 200 µl tips. Migration of the cells into the wound was recorded 8 hours later, unless otherwise specified.

Statistical analysis: Results are expressed as mean ± SEM values of the indicated number of independent experiments (n). Origin software was used for statistical data analysis. Statistical significance was determined by Student's t-test for paired comparison. P < 0.05 was considered significant.

### Example 1: A high throughput screening for GTPase activity of recombinant OPA1.

Since the three-dimensional crystal structure of OPA1 is unavailable, was first built a structural model based on the homology of OPA1 with twenty protein templates selected to maximize confidence, percentage identity and alignment coverage of OPA1. Inspection of said model revealed an overall structural organization resembling that of crystallized proteins of the family, including dynamin and Drp1 REF. It revealed the organization of the GTPase domain, with the position of residues unique to Opal that can confer OPA1 specificity over other members of the superfamily. In order to identify lead molecules that could inhibit Opal, we turned to a wet biology approach, the same inventors scaled up Ni-NTA beads affinity chromatography protein purification, yielding approx. XXX mg of rOPA1 per L of BL(DE3) E. coli. Km and Vmax of the dialyzed rOPA1 measured by reverse phase chromatography (RPC) were 274.1±20.5 µM and 0.75±0.18, respectively. Because reverse phase chromatography is unsuitable for high throughput analysis, the same inventors turned to the malachite green (MG) colorimetric assay that quantifies the free phosphate released during the hydrolysis of GTP into GDP. Kinetic properties of rOPA1 measured with the MG assay were superimposable to those obtained by RPC, further supporting the use of this approach to screen for OPA1 inhibitors. The same inventors adapted the assay to a high-throughput mode, by first optimize the enzyme to substrate ratio (rOPA1/GTP) to increase the sensitivity of the miniaturized assay without affecting the specificity of the detection and then fixing rOPA1 at 0.2 µM and GTP at 500 µM. Next, the same inventors confirmed that rOPA1 activity could be inhibited by increasing concentrations of the non-hydrolysable GTP analogues GTPγS and GMPPNP. They also evaluated the potential interference of dimethyl sulfoxide (DMSO), the solvent used to solubilize the compounds from the chosen library, on measured rOPA1 activity, and excluded that a final 2.5% (V/V) DMSO concentration affected the measured rOPA1 activity or interfered with MG absorbance. The same inventors then looked for a strong positive control for rOPA1 inhibition. We compared inhibition by GTPγS and ethylenediaminetetraacetic acid (EDTA) which chelates the Mg²⁺ ions required for GTP hydrolysis. While they retrieved a residual rOPA1 activity upon GTPγS treatment, EDTA stably and fully inhibited GTP hydrolysis. Moreover, GTPγS strongly altered the absorbance readout, further complicating its use in a high-throughput setting. To understand whether rOPA1 inhibition was affected by the duration of incubation time with the inhibitor before GTP addition, the same inventors assessed the influence of different GTPγS incubation times on rOPA1 activity. Although no significant difference was observed, incubation for 10 minutes appeared to be enough to fully inhibit the enzyme, in line with the general suggestion that equilibration between enzyme and the potential inhibitor prior to addition of the substrate shall be allowed (Copeland, R.A., 2003. Mechanistic considerations in high-throughput screening. Analytical Biochemistry 320, 1-12). Finally, the same inventors we optimized buffer conditions for rOPA1 GTPase activity measurements (Quintana-Cabrera, R., Quirin, C., Glytsou, C., Corrado, M., Urbani, A., Pellattiero, A., Calvo,E., Vazquez, J., Enriquez, J.A., Gerle, C., et al. 2018. The cristae modulator Optic atrophy 1 requires mitochondrial ATP synthase oligomers to safeguard mitochondrial function Nature communications 9, 3399). Like the other dynamin superfamily GTPases, rOPA1 required Mg²⁺ as cofactor for GTP hydrolysis, which was irreplaceable by Mn²⁺ and a low ionic strength buffer to prevent the protein oligomerization and precipitation. Furthermore, rOPA1 GTPase activity was not influenced by 500 µM GDP, excluding inhibition by GDP throughout the incubation time of the assay. These parameters were used to design a pipeline for a semi-automated 384-well plates HTS, as shown in the following table 1, wherein Assay format: 384-well plates, Assay volume/well = 20 µl, Final concentrations/well: 0.5 µM rOPA1, 500 µM GTP, 10 mM MgCl₂, 2.5% DMSO.

**Table 1**

| **Step** | **Parameter** | **Value** | **Description** |
|---|---|---|---|
| 1 | *Library compounds* | 5 µl | From 200 µM to 830 µM stock compounds |
| 2 | *rOPA1* | 8 µl | 1.2 µM stock |
| 3 | *Incubation time* | 10 min | Room temperature |
| 4 | *Positive controls* | 5 µl | 0.5 M EDTA stock, pH 8.0 |
| 5 | *Start reaction* | 7 µl | 1.43 mM GTP stock, 28.6 mM MgCl₂ stock |
| 6 | *Incubation time* | 2 h | 37°C |
| 7 | *Stop reaction* | 5 µl | 0.5 M EDTA stock, pH 8.0 |
| 8 | *Reporter reagent* | 60 µl | 1.5 mM malachite green stock, 8 mM ammonium molybdate stock |
| 9 | *Incubation time* | 15 min | Room temperature |
| 10 | *Assay readout* | 595 nm | Microplate reader, absorbance mode |

| **Step** | **Notes** | | |
|---|---|---|---|
| 1 | Library "MyriaScreen" (Aldrich) of 10,000 drug-like small molecule dissolved in 10% DMSO. | | |
| 2 | rOPA1 correspondent to Mus Musculus isoform 1 (Uniprot: P58281) expressed and purified from E. coli. | | |
| 3 | Plates lidded to allow compound-rOPA1 interaction. | | |
| 4 | EDTA chelates Mg²⁺ that cannot be used for rOPA1 GTP hydrolysis reaction. | | |
| 5 | Fresh solutions mixed just before use. | | |
| 6 | Plates are sealed to avoid evaporation and leaved in an incubator. | | |
| 7 | Add to all the wells excluding positive control wells. EDTA chelates Mg²⁺ stopping the GTP hydrolysis reaction. | | |
| 8 | Malachite green and ammonium sulphate are stored at 4°C in the dark and mixed just before use. Mix gently by tapping the plate. | | |
| 9 | Plates lidded until color development. | | |

### Example 2: HTS for rOPA1 small molecule inhibitors, data-processing and hit identification

MG based, rOPA1 GTPase activity HTS, was performed on a library of 10,000 selected, pure drug-like small molecules (MyriaScreen, Aldrich) enriched with privileged motifs computed to fulfill the "Lipinski's rule of 5", which defines the essential chemical and physical properties of drugs to ensure their permeability and bioavailability (Lipinski, C.A., Lombardo, F., Dominy, B.W., and Feeney, P.J. (2001). Experimental and computational approaches to estimate solubility and permeability in drug discovery and development settings. Adv Drug Deliv Rev 46, 3-26). The 10,000 small compounds of the MyriaScreen library were tested at a single concentration ranging from 50 to 200 µM. Because high-throughput assays may show large variability with respect to intra-plate and inter-plate results, day-to-day and batch-to-batch comparisons (Goktug, A.N., Chai, S.C., and Chen, T. (2013). Data Analysis Approaches in High Throughput Screening), graphical and statistical quality control metrics was used to validate the quality of the assay and to meet minimum standards ensuring that positive hits can be distinguished from the noise of the assay.

Strictly standardized mean difference [SSMD], i.e. the ratio of the mean to the standard deviation (SD) of the difference between the two populations was used, it measures the strength of the difference between two compared groups, to assess the difference between positive and the negative control samples. The larger the value of the SSMD between the two controls, the greater the difference between the two populations. In assays resembling ours, where positive controls (maximal inhibition of rOPA1) have a value smaller than negative controls (minimal inhibition of OPAl), plates with SSMD values ≤-3 pass the quality control (Zhang, X.D. (2007). A pair of new statistical parameters for quality control in RNA interference high-throughput screening assays. Genomics 89, 552-561). In the present case the assay remained sufficiently robust throughout the screening and, as demonstrated by the plate-specific SSMD values, only 4 plates did not pass quality control. The graphical raw data visualization also contributes to the quality control review process. Raw data of each sample of the 32 assayed 384-well plates against its well number were plotted. The resulting scatter plot indicated that the assay was well behaved. With few exceptions, an average robust assay signal window between positive and negative controls was identified, even if negative controls spanned a larger range of values than the positive controls. Also, the scatter plot revealed that the 10,000 small-molecules could be divided in two clearly distinct populations. Most of the compounds were inactive, i.e. their effect on rOPA1 activity resembled that of the negative controls. A second population comprised active compounds, whose rOPA1 inhibition levels falling in between negative and positive controls. The same inventors normalized the MG raw data as normalized percent of inhibition (NPI), i.e. as percentage activity relative to negative controls and then binned the NPI values in a corresponding trellis heat map of the 32 screened 384-well plates, where wells were colored according to the binning, encoded by continuous color changes from dark red (high inhibition) to dark blue (low inhibition). The heat map did not reveal any noticeable systematic pattern that may represent error of reagent pipetting. In both the heat map and the scatter plot representations, control and sample areas matched with respect to the raw measurement values. A more careful inspection allowed to identify the 4 excluded plates with lower variability in the positive and negative controls, already readily apparent in the SSMD plot. Nonetheless, these 4 plates did not exhibit significantly different patterns in the distribution of the NPI values of the compounds. Due to the low activity of rOPA1 and the assay detection limits, the primary screen was performed with relatively high GTP concentrations, as 500 µM GTP is above the Km of OPA1 (Quintana-Cabrera, R., Quirin, C., Glytsou, C., Corrado, M., Urbani, A., Pellattiero, A., Calvo, E., Vazquez, J., Enriquez, J.A., Gerle, C., et al. (2018). The cristae modulator Optic atrophy 1 requires mitochondrial ATP synthase oligomers to safeguard mitochondrial function, Nature communications 9, 3399). Generally, this enriches the identification of uncompetitive compounds and non-competitive inhibitors instead of competitive inhibitors (Copeland, R.A. (2003). Mechanistic considerations in high-throughput screening. Analytical Biochemistry 320, 1-12). Running the assay at higher substrate concentrations was however inevitable to obtain a sufficiently robust assay. Yet, the conditions were merely met, and we compensated for the assay's limitations by setting a conservative threshold that included only the most potent inhibitors of the screen. The calculated NPI values from the raw data allowed ranking the samples based on their inhibitory potential. Most of the tested compounds showed no effect on OPA1 GTPase activity, as shown before, and hence had a value around 1, similar to the negative controls. Compounds that affected OPA1 activity displayed values smaller than 1 with a minimum of 0, as the positive control containing EDTA. Considering the compounds with a NPI value between 1 and 0, we selected as inhibitors the compounds that decreased the activity of OPA1 by at least 30%, corresponding to NPI < 0.7. After two rounds of library screening, only compounds identified in both individual and replicate analysis were retained. Of these, the compounds that per se reduced the absorbance at the measured wavelength were considered as false positives and were excluded from the list. Eventually, we identified 8 potential inhibitor molecules that decreased OPA1 GTPase activity by at least 30% and that were christened MYLS2, MYLS3, MYLS5, MYLS9, MYLS10, MYLS14, MYLS16 and MYLS22, as shown in figure 11.

### Example 3: Hit validation by concentration response curve

The screened library contains small compounds selected based on their drug-likeness and for their chemical diversity. Hence, all identified compounds fulfilled the Lipinski's rule of 5 (Lipinski, C.A., Lombardo, F., Dominy, B.W., and Feeney, P.J. (2001). Experimental and computational approaches to estimate solubility and permeability in drug discovery and development settings. Adv Drug Deliv Rev 46, 3-26). Only MYLS21, also known as Suramin, is an exception to these rules, yet it is the only compound already in the clinics (Kaur et al., 2002). To evaluate the 8 compounds selected from the primary HTS for their ability to inhibit rOPA1 GTPase activity, they were tested in a dose-response experiment at concentrations ranging from 1 nM to 750 µM. All 8 inhibitors showed an inhibitory activity increasing with the rising of their concentrations (Figures 6-10), confirming that they truly inhibit rOPA1 GTPase activity. Data were fitted with a four-parameter curve to evaluate the potency as half-maximal inhibitory concentration (IC₅₀) and maximal inhibition (Iₘₐₓ), as well as the nature of the inhibition, based on the calculation of Hill coefficient (Figures 6-10). The compounds were grouped into moderate (Figures 7,9,10) and potent inhibitors (Figures 6,8) according to the maximal rOPA1 inhibition they exerted. Compounds that bind a single site on an enzyme yield Hill slopes of 1 and deviations may indicate that the inhibitor blocks activity nonspecifically. Most of the identified inhibitors showed a Hill slope bigger than 1 except for MYLS5, MYLS22 and MYLS16, which may suggest unspecific inhibition through aggregation. In conclusion, the first in class in vitro inhibitors of OPA1 GTPase activity was identified. Among them MYLS22 was recognized as the most potent inhibitor of OPA1 activity (IC50=96.5 µM, Iₘₐₓ=0.78) (Figure 11).

### Example 4: MYLS22 is a specific OPA1 GTPase activity inhibitor.

To identify possible MYLS22-binding sites within OPA1 structure and to confirm the results of the in vitro screening, a molecular docking analysis was set up. Interestingly, the best scoring pose of MYLS22 was identified within the hydrophobic GTP-binding pocket of OPA1 (Figure 2). A close analysis of the MYLS22 binding pose suggested hydrogen bonding interactions with the residues Gln-297, Gly-319 and Asn-404 in the active site of OPA1 (Figures 1,2). Gln-297 and Gly-319 are two well conserved residues in the P-loop and in the Switch I regions of dynamins, that participate in the GTP hydrolysis process of OPA1 and show a high degree of similarity with the other dynamin superfamily proteins. Notably, beyond the GTP-binding motif, MYLS22 also potentially contacts an OPAl-specific region that include Asn-404 and does not present similarity with other dynamins. This interaction appears to be important for defining potential specificity of the inhibitor. Even though the in silico docking results suggested some potential specificity of OPA1 inhibition by MYLS22, GTPase domain is the most conserved region among the dynamin superfamily proteins. Therefore the specificity of MYLS22 toward OPA1 was tested by comparing the effects of the identified OPA1 inhibitors on the GTPase activity of Dynamin-1 (Dyn1), one of the closest homologues of OPA1 (Ford, M.G., Jenni, S., and Nunnari, J. (2011). The crystal structure of dynamin. Nature 477, 561-566). Using the MG assay, the GTPase activity of a recombinant Dynamin-1 lacking the proline rich domain (PRD) (Dyn1ΔPRD) in the presence of MYLS22 was measured. While the other identified inhibitors displayed different degrees of Dyn1ΔPRD inhibition, MYLS22 did not, even at concentrations four times above the calculated IC50 for OPA1. In conclusion, MYLS22 binds to OPA1 GTPase domain and selectively inhibits OPA1 GTPase activity.

### Example 5: MYLS22 is a cell-permeable lipophilic small molecule.

The determination of the cellular bioavailability of small-molecule inhibitors is a critical step for interpreting cell-based data and directing inhibitor optimization (Teuscher, K.B., Zhang, M., and Ji, H. (2017). A Versatile Method to Determine the Cellular Bioavailability of Small-Molecule Inhibitors). The discrepancies between the results obtained in biochemical and cell-based assays have been a recurring problem when novel inhibitors identified from biochemical studies are assessed in cells (Bunnage et al., 2013 Bunnage, M.E., Chekler, E.L., and Jones, L.H. (2013). Target validation using chemical probes. Nat Chem Biol 9, 195-199; Hann, M.M., and Simpson, G.L. (2014). Intracellular drug concentration and disposition--themissing link? Methods 68, 283-285; Morgan, P., Van Der Graaf, P.H., Arrowsmith, J., Feltner, D.E., Drummond, K.S., Wegner, C.D., and Street, S.D. (2012). Can the flow of medicines be improved? Fundamental pharmacokinetic and pharmacological principles toward improving Phase II survival. Drug Discov Today 17, 419-424). This difference has traditionally been ascribed to the low permeability of the compound through the cell membrane. Thus, MYLS22 physicochemical properties of MYLS22 were first assessed, which included its aqueous solubility and stability in cell culture medium. MYLS22 is a drug-like molecule that meets all the physiochemical requirement indicated in the Lipinski's rule of five. Nevertheless, the inhibitor is characterized by a low aqueous solubility and a low hydrophilicity (Log P = 3.67, Log D = 3.51) (Table 1). MYLS22 is soluble in DMSO up to 1 mM and in cell culture medium up to 150µM. Fluorescence provides a sensitive mean to assess whether a drug can be taken up by cells, but its use is limited by the occurrence of a chromophore that can absorb UV or visible light providing fluorescence at a longer wavelength. MYLS22 is characterized by absorption and emission bands in the UV region of the electromagnetic spectrum and, unfortunately, it does not have any chemical structure that produces a fluorescence signal. Therefore, the fluorescence properties of the molecule can not be used for its direct visualization in complex media. The cell permeability of MYLS22 with two independent and complementary methods by exploiting its absorbance at 280 nm, was measured. Mouse embryonic fibroblasts (MEFs) were incubated with MYLS22 in HBSS, to avoid the strong 280nm absorption by proteins of DMEM and the remaining MYLS22 was measured (by using its 280nm absorption as a proxy) over time. The concentration of MYLS22 in the extracellular medium decreased with incubation time. However, this did not exclude its non-specific binding to the culture dish. To verify the effective presence of MYLS22 inside the cells, was performed a reversed-phase high-performance liquid chromatography (HPLC)-UV method on lysates preprocessed with solid-phase extraction (SPE), which significantly enhanced the sensitivity of the HPLC-UV method. The SPE led to the double advantage of separating small hydrophobic molecules from cell lysate proteins and of simultaneously changing the solvent, thus improving MYLS22 solubility. After SPE, samples were injected into an analytical reversed-phase C18 column with a gradient of trifluoroacetic acid (TFA) (0.1% v/v)/acetonitrile as mobile phase. This method allowed us to distinctly isolate the elution peak of MYLS22 inhibitor detected by the absorbance at 280 nm, in a total run time of 15 min. Overall, these results demonstrated that MYLS22 can enter mammalian cells.

### Example 6: MYLS22 per se does not cause cell death and is not mitochondriotoxic.

Then was verified if MYLS22 induced direct cytotoxic effects. Concentrations of MYLS22 up to 100 µM did not cause MEFs death (Figure 3). Because cultured cells can survive extensive mitochondrial dysfunction by operating the reversal mode of the ATP synthase, was measured if MYLS22 stimulated mitochondrial depolarization in response to the F1F0 ATPase inhibitor oligomycin, a sensitive test of latent mitochondrial dysfunction in intact cells (Irwin, W.A., Bergamin, N., Sabatelli, P., Reggiani, C., Megighian, A., Merlini, L., Braghetta, P., Columbaro, M., Volpin, D., Bressan, G.M., et al. (2003). Mitochondrial dysfunction and apoptosis in myopathic mice with collagen VI deficiency. Nat Genet 35, 367-371). Real-time imaging of the mitochondrial potentiometric dye tetramethyl rhodamine methyl ester (TMRM) fluorescence in response to oligomycin showed that membrane potential was stable in MEFs treated with MYLS22 over a range of concentrations well above rOPA1 IC50. Lack of mitochondrial toxicity in vitro was confirmed by using mitochondria purified from mouse liver. Irrespective of whether mitochondria were energized using complex I (glutamate/malate) or complex II (succinate) substrates, MYLS22 pretreatment did not reduce uptake of the potentiometric dye Rhodamine 123, over a range of concentrations well above rOPA1 IC50. Thus, MYLS22 does not induce per se primary mitochondrial dysfunction and was defined a safe range of MYLS22 concentrations that could be used to assess inhibitor-specific cellular function devoid of unrelated cytotoxic side effects.

### Example 7: MYLS22 induces mitochondrial fragmentation.

OPA1 is a mitochondrial-shaping protein involved in inner mitochondrial membrane fusion and as expected, Opa1-/- cells display fragmented mitochondrial network with defects in cristae shape. Notably, OPA1 requires an intact GTPase domain to fuse mitochondria and GTPase mutants expressed in cells harboring WT OPA1 cause mitochondrial fragmentation. Because of these aspects of OPA1 biology, any chemical OPA1 inhibitor is expected to cause mitochondrial fragmentation. Therefore was tested whether a panel of MYLS22 concentrations led to fragmentation of WT MEFs mitochondria. Visual inspection of confocal images of MEFs expressing mitochondrially targeted YFP (mtYFP) revealed that MYLS22 caused a dose dependent mitochondrial fragmentation and decrease in mitochondrial length, leading to the appearance of completely fragmented organelles similar to the ones imaged in Opal -/- MEFs. In conclusion, MYLS22 causes extensive mitochondrial fission in situ.

### Example 8: MYLS22 enhances cytochrome c release from mitochondria during apoptosis.

In addition to, and independently from its role in mitochondrial fusion, OPA1 plays a key function in the regulation of apoptotic cell death. Its downregulation increases cell sensitivity to induced apoptosis. Because OPA1 requires integrity of the GTPase domain also to prevent cytochrome c release, OPA1 inhibition by MYLS22 leading to enhanced cytochrome c release was expected. Indeed, MYLS22 enhanced in a concentration-dependent manner the amount of cytochrome c released from purified mouse liver mitochondria in response to the active form of the proapoptotic Bcl2 molecule BID (cBID), irrespective of whether we measured it by immunoblotting or by an established quantitative cytochrome c ELISA. Already at a low concentration of 25µM, MYLS22 increased by 1.7-fold the amount of cytochrome c release, that was more than doubled when mitochondria were treated with 125 µM MYLS22. Interestingly, these two MYLS22 concentrations enhanced the cBID-mediated disruption of the chemically crosslinkable OPA1 oligomers, whose destabilization correlates with cristae remodeling and complete cytochrome c release.

Then was verified whether MYLS22 similarly influenced the release of cytochrome c in a cell-based assay. Following treatment with the intrinsic apoptotic, mitochondria dependent stimulus H₂O₂, MEFs incubated with 50µM MYLS22 released cytochrome c significantly more rapidly than cells incubated with 25 µM MYLS22 or with DMSO, reaching maximal release already 30 min after apoptosis induction (Figure 6). Taken together, these data show that MYLS22 treatment enhances in a dose-dependent manner the release of cytochrome c in response to an apoptotic stimulus.

### Example 9: breast cancer cells migration is sensitive to genetic and pharmacological inhibition of OPA1.

Previous data data confirm that processes controlled by OPA1 (mitochondrial fusion and cytochrome c release) can be targeted by OPA1 inhibitors in vivo, they have been obtained on normal mouse organelles and tissues. MDA-MB-231 cells, a human metastatic breast adenocarcinoma cell line were used for measuring if MYLS22 could block MDA-MB-231 migration, an essential feature of cancer metastatization that in these cells is controlled by mitochondrial morphology (Zhao, J., Zhang, J., Yu, M., Xie, Y., Huang, Y., Wolff, D.W., Abel, P.W., and Tu, Y. (2012). Mitochondrial dynamics regulates migration and invasion of breast cancer cells. Oncogene 32, 4814). When OPA1 was blocked genetically, by efficient siRNA-mediated silencing of OPA1 migration was reduced by around 50% in a classic scratch assay. A very similar inhibition was achieved by treatment with MYLS22. Most importantly, the effect of MYLS22 on cell migration was not additive to that of OPA1 silencing, indicating that MYLS22 acts specifically by blocking OPA1. Overall, these results demonstrate that safe concentrations of MYLS22 decrease cancer cell migration and suggest that MYLS22 act on the same OPAl-dependent pathway that controls cell migration.

In the following table 2 are summarized the physiochemical properties of MYLS22.

**Table 2**

| | Calculated values for Inh#22 | Desirable values |
|---|---|---|
| Molecular Weight | 443.53 Da | < 500 Da |
| pl | 6.12 | - |
| Log P | 3.67 | - |
| Log D (pH 7.4) | 3.51 | < 5 |
| H-donors | 1 | < 5 |
| H-acceptors (pH 7.4) | 6 (< 10) | < 10 |
| Rotatable bounds | 1 | < 10 |
| Molar Refractivity | 125.25 | from 40 to 130 |
| Hydrophilic-lipophilic balance number (HLB) | 8.05 (Davies) | - |
| | 11.11 (Griffin) | |
| | 9.27 (combined method) | |
| Intrinsic solubility (Log S) (pH 7.4) | -5.72 | from 30 to 130 |
| Polar surface area (pH 7.4) | 70.47 Å | < 140 Å |
| Number of atoms | 50 | from 20 to 70 |

## Claims

1. Pharmaceutical composition comprising a therapeutically effective amount of at least one compound of formula (I): 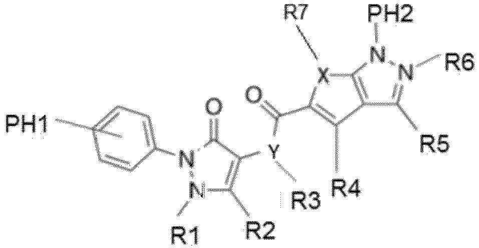 wherein
X and Y, the same or different, is N,O,S,B,P
PH1 and PH2, the same or different, is phenyl,
optionally substituted with H, OH, halogen, linear or branched saturated or unsaturated C1-C20 alkyl, optionally substituted by one or more amino, imino, hydroxyl and aldehyde groups, and/or can contain one or more nitrogen groups, optionally substituted by a C1-C6 linear or branched, saturated or unsaturated alkyl group;
R9C=CR13, R9C≡CR13, R9(Halogen), R9OH, R9COR13, R9CHO, R9CO(halogen), R90COOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9)(OR13) (OR14) (OR15), R9(CO)O(CO)R14, CO(R9)₂, , CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO- (CH₂)ₙ-COOH, CH=NO-(CH₂)ₙ-NH₂, CH=NO-(CH₂)ₙ-OH, (CH₂)ₙNHCO-R9, (CH₂)ₙ-R9, CH=NOR9, CH₂OH, (CH₂)ₙNH-R9, (CH₂)ₙN(R9) ₂, (CH₂)ₙ-R10, C(=NH)NH₂, C≡CCOOH CC≡CCH₂OH, C≡CC(R9)₂OH, C≡CCOO-R9, C≡CCONH₂, C≡CONH-R9,
C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS,
R1, R2, R3, R4,R5, R6, R7, the same or different, is H, OH, halogen, optionally substituted by one or more amino, imino, hydroxyl and aldehyde groups, and/or can contain one or more nitrogen groups, optionally substituted by a C1-C6 linear or branched, saturated or unsaturated alkyl group, CHO, NH₂, R9C=CR13, R9C≡CR13, R9(Halogen), R9OH, R9COR13, R9CHO, R9CO(halogen), R90COOR13, R9COOH, R9COOR13, R9OCH₃, R9OOH, R9OOR13, R90R13, R9CH(OR13) (OH), R9C(OR14) (OH)R13, R9CH(OR13) (OR14), R9C(OR13) (OR14)R15, R9C(OR13) (OR14) (OR15), C(OR9) (OR13) (OR14) (OR15), R9(CO)O(CO)R14, CO(R9)₂, , CO-R9, OR9,COOH-R9, NH₂, NHOH, NHNH₂, NH-R9, NH(R9)₂, CH(halogen)₃, COC(halogen)₃, (CH₂)ₙ-R8 CH=NOH, CH=NO-R9, CH=NO- (CH₂)ₙ-COOH, CH=NO-(CH₂)ₙ-NH₂, CH=NO-(CH₂)ₙ-OH, (CH₂)ₙNHCO-R9, (CH₂)ₙ-R9, CH=NOR9, CH₂OH, (CH₂)ₙNH-R9, (CH₂)ₙN(R9)₂, (CH₂)ₙ-R10, C(=NH)NH₂, C≡CCOOH CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9,SCN, CSR9, R9C=OS, phenyl, aryl, bicyclic, heterocyclic
R8 is H, OH, COOH, NH₂,
R9,R13,R14 is, the same or different, H, linear or branched saturated or unsaturated C₁-C₂₀ alkyl
n is an integer n is an integer from 1 to 20
R10 is OH, NH₂, N-R11R12,
R11 and R12, the same or different is H, C₁-C₁₀ alkyl linear or branched saturated or unsaturated,
their enantiomeric and/or diastereomeric mixtures, their pharmaceutically acceptable salts, their solvates and hydrates, for use in the treatment of cancer.

2. Pharmaceutical composition for use according to claim 1 wherein in the compound of formula (I) PH1 and PH2 are phenyl, R1 R2 and R5 are CH₃, R6 R7 are H, X is S, Y is N.

3. Pharmaceutical composition for use according to claim 2 wherein the compound of formula (I) is N-(1,5-dimethyl-3-oxo-2-phenyl-2,3-dihydro-1H-pyrazol-4-YL)-3-methyl-1-phenyl-1H-thieno[2,3-c]pyrazole-5-carboxamide. (C24H21N5O2S).

4. Pharmaceutical composition for use according to claim 1 wherein the pharmaceutical composition further comprises a chemotherapeutic drug.

5. Pharmaceutical composition for use according to claim 4 wherein the chemotherapeutic drug is selected from the group consisting of doxorubicin, etoposide and cisplatin.

6. Pharmaceutical composition for use according to claim 1 wherein cancer is breast cancer, lung cancer.

## Patentansprüche

1. Pharmazeutische Zusammensetzung umfassend eine therapeutisch wirksame Menge wenigstens einer Verbindung der Formel (I): 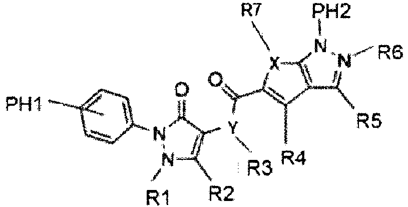 worin
X und Y, gleich oder verschieden, N, O, S, B, P ist
PH1 und PH2, gleich oder verschieden, Phenyl ist,
optional substituiert mit H, OH, Halogen, linearem oder verzweigtem gesättigten oder ungesättigtem C1-C20-Alkyl, optional substituiert mit einer oder mehr Amino-, Imino-, Hydroxyl- und Aldehyd-Gruppen und/oder kann eine oder mehr Stickstoffgruppen enthalten, optional substituiert mit einer linearen oder verzweigten, gesättigten oder ungesättigten C1-C6-Alkylgruppe;
R9C=CR13, R9C=CR13, R9(Halogen), R9OH, R9COR13, R9CHO, R9CO(Halogen), R9OCOOR13, R9COOH, R9COOR13, R9OCH₃, R9OOH, R9OOR13, R9OR13, R9CH(OR13)(OH), R9C(OR14)(OH)R13, R9CH(OR13)(OR14), R9C(OR13)(OR14)R15, R9C(OR13)(OR14)(OR15), C(OR9)(OR13)(OR14)(OR15), R9(CO)O(CO)R14, CO(R9)₂, CO-R9, O-R9, COOH-R9, NH₂, NHOH, NHNH₂, NH-R9, NH(R9)₂, CH(Halogen)₃, COC(Halogen)₃, (CH₂)ₙ-R8, CH=NOH, CH=NO-R9, CH=NO-(CH₂)ₙ-COOH, CH=NO-(CH₂)ₙ-NH₂, CH=NO-(CH₂)ₙ-OH, (CH₂)ₙNHCO-R9, (CH₂)ₙ-R9 CH=NO-R9, CH₂OH, (CH₂)ₙNH-R9, (CH₂)ₙN(R9)₂, (CH₂)ₙ₋R10, C(=NH)NH₂, C≡CCOOH, CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9,SO₂R9, SCN, CSR9, R9C=OS,
R1, R2, R3, R4,R5, R6, R7, gleich oder verschieden, ist H, OH, Halogen, optional substituiert mit einer oder mehr Amino-, Imino-, Hydroxyl- und Aldehydgruppen, und/oder kann eine oder mehr Stickstoffgruppen enthalten, optional substituiert mit einer linearen oder verweigten, gesättigten oder ungesättigten C1-C6-Alkylgruppe, CHO, NH₂, R9C=CR13, R9C=CR13, R9(Halogen), R9OH, R9COR13, R9CHO, R9CO(Halogen), R9OCOOR13, R9COOH, R9COOR13, R9OCH₃, R9OOH, R9OOR13, R9OR13, R9CH(OR13)(OH), R9C (OR14)(OH)R13, R9CH(OR13)(OR14),
R9C(OR13)(OR14)R15, R9C(OR13)(OR14)(OR15), C(OR9)(OR13)(OR14)(OR15), R9(CO)O(CO)R14, CO(R9)₂, CO-R9, OR9, COOH-R9, NH₂, NHOH, NHNH₂, NH-R9, NH (R9)₂, CH(Halogen)₃, COC(Halogen)₃,
(CH₂)ₙ-R8, CH=NOH, CH=NO-R9, CH=NO-(CH₂)ₙ-COOH, CH=NO-(CH₂)ₙ-NH₂, CH=NO-(CH₂)ₙ-OH, (CH₂)ₙNHCO-R9, (CH₂)n-R9, CH=NO-R9, CH₂OH, (CH₂)ₙ NH-R9, (CH₂)ₙN(R9)₂, (CH₂)ₙ₋R10, C(=NH)NH₂, C=CCOOH, CC≡CCH₂OH, C≡CC(R9)₂OH, C≡CCOO-R9, C≡CCONH₂, C≡CONH-R9, C≡CCONHOH, NH-R9, N(R9)₂, CONH-R9-S-S-R9, CONH-R9-S-S-alkyl-NH₂, R9S, R9SR13, SOR9, SO₂R9, SCN, CSR9, R9C=OS, Phenyl, Aryl, bizyklisch, heterozyklisch
R8 ist H, OH, COOH, NH₂,
R9, R13, R14 ist, gleich oder verschieden, H, lineares oder verzweigtes gesättigtes oder ungesättigtes C₁-C₂₀-Alkyl
n ist eine ganze Zahl n ist eine ganze Zahl von 1 bis 20
R10 ist OH, NH₂, N-R11R12,
R11 und R12, gleich oder verschieden, ist H, C₁-C₁₀-Alkyl, linear oder verzweigt, gesättigt oder ungesättigt,
ihre enantiomeren und/oder diasteromeren Mischungen, ihre pharmazeutisch verträglichen Salze, ihre Solvate und Hydrate, zur Verwendung bei der Behandlung von Krebs.

2. Pharmazeutische Zusammensetzung zur Verwendung gemäß Anspruch 1, worin bei der Verbindung der Formel (I) PH1 und PH2 Phenyl sind, R1, R2 und R5 CH₃ sind, R6, R7 H sind, X S ist, Y N ist.

3. Pharmazeutische Zusammensetzung zur Verwendung gemäß Anspruch 2, worin die Verbindung der Formel (I) N-(1,5-Dimethyl-3-oxo-2-phenyl-2,3-dihydro-1H-pyrazol-4-YL)-3-methyl-1-phenyl-1H-thieno[2,3-c]pyrazol-5-carboxamid ist. (C24H21N5O2S).

4. Pharmazeutische Zusammensetzung zur Verwendung gemäß Anspruch 1, worin die pharmazeutische Zusammensetzung weiterhin ein chemotherapeutisches Arzneimittel umfasst.

5. Pharmazeutische Zusammensetzung zur Verwendung gemäß Anspruch 4, worin das chemotherapeutische Arzneimittel ausgewählt ist aus der Gruppe bestehend aus Doxorubicin, Etoposid und Cisplatin.

6. Pharmazeutische Zusammensetzung zur Verwendung gemäß Anspruch 1, worin der Krebs Brustkrebs, Lungenkrebs ist.

## Revendications

1. Composition pharmaceutique comprenant une quantité thérapeutiquement efficace d'au moins un composé de formule (I) : 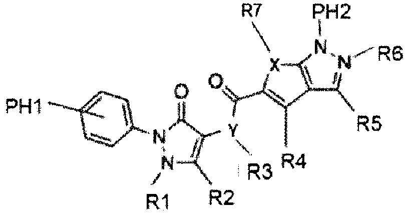 dans laquelle
X et Y, identiques ou différents, sont N, O, S, B, P
PH1 et PH2, identiques ou différents, sont des phényles,
éventuellement substitués par H, OH, un halogène, un alkyle linéaire ou ramifié, saturé ou insaturé en C1-C20, éventuellement substitués par un ou plusieurs groupes amino, imino, hydroxyle et aldéhyde, et/ou peuvent contenir un ou plusieurs groupes d'azote, éventuellement substitués par un groupe alkyle linéaire ou ramifié, saturé ou insaturé en C1-C6 ;
R9C=CR13, R9C=CR13, R9(Halogène), R9OH, R9COR13, R9CHO, R9CO(halogène), ROOCOOR13, R9COOH, R9COOR13, R9OCH₃, R9OOH, R9OOR13, R9OR13, R9CH(OR13)(OH), R9C(OR14)(OH)R13, R9CH(OR13)(OR14), R9C(OR13)(OR14)R15, R9C(OR13)(OR14)(OR15), C(OR9)(OR13)(OR14)(OR15), R9(CO)O(CO)R14, CO(R9)₂, CO-R9, O-R9, COOH-R9, NH₂, NHOH, NHNH₂, NH-R9, NH(R9)₂, CH(halogène)₃, COC(halogène)₃, (CH₂)ₙ-R8, CH=NOH, CH=NO-R9, CH=NO-(CH₂)ₙ-COOH, CH=NO-(CH₂)ₙ-NH₂, CH=NO-(CH₂)ₙ-OH, (CH₂)ₙNHCO-R9, (CH₂)ₙ-R9, CH=NO-R9, CH₂OH, (CH₂)ₙNH-R9, (CH₂)ₙN(R9)₂, (CH₂)ₙ-R10, C(=NH)NH₂, C=CCOOH, CC=CCH₂OH, C=CC(R9)₂OH, C=CCOO-R9, C=CCONH₂, C=CONH-R9, C=CCONHOH, NH-R9, N(R9)₂, CONH-R9-S-S-R9, CONH-R9-S-S-alkyl-NH₂, R9S, R9SR13, SOR9, SO₂R9, SCN, CSR9, R9C=OS,
R1, R2, R3, R4, R5, R6, R7, identiques ou différents, sont H, OH, un halogène, éventuellement substitués par un ou plusieurs groupes amino, imino, hydroxyle et aldéhyde, et/ou peuvent contenir un ou plusieurs groupes d'azote, éventuellement substitués par un groupe alkyle en C1-C6, linéaire ou ramifié, saturé ou insaturé, CHO, NH₂, R9C=CR13, R9C=CR13, R9(Halogène), R9OH, R9COR13, R9CHO, R9CO(halogène), R9OCOOR13, R9COOH, R9COOR13,
R9OCH3, R9OOH, R9OOR13, R9OR13, R9CH(OR13)(OH), R9C(OR14)(OH)R13, R9CH(OR13)(OR14), R9C(OR13)(OR14)R15, R9C(OR13)(OR14)(OR15), C(OR9)(OR13)(OR14)(OR15), R9(CO)O(CO)R14, CO(R9)₂, CO-R9, O-R9, COOH-R9, NH₂, NHOH, NH-NH₂, NH-R9, NH(R9)₂, CH(halogène)₃, COC(halogène)₃, (CH₂)ₙ-R8, CH=NOH, CH=NO-R9, CH=NO-(CH₂)ₙ-NH₂, CH=NO-(CH₂)ₙ-OH, (CH₂)ₙNHCO-R9, (CH₂)n-R9, CH=NO-R9, CH₂OH, (CH₂)ₙNH-R9, (CH₂)ₙN(R9)₂, (CH₂)ₙ-R10, C(=NH)NH₂, C=CCOOH, CC=CCH₂OH, C=CC(R9)₂OH, C=CCOO-R9, C=CCONH₂, C=CONH-R9, C=CCONHOH, NH-R9, N(R9)₂, CONH-R9-S-S-R9, CONH-R9-S-S-alkyl-NH₂, R9S, R9SR13, SOR9, SO₂R9, SCN, CSR9, R9C=OS, un phényle, aryle, bicyclique, hétérocyclique
R8 est H, OH, COOH, NH₂,
R9, R13, R14 sont, identiques ou différents, H, un alkyle linéaire ou ramifié, saturé ou insaturé en C₁-C₂₀
n est un nombre entier, n est un nombre entier de 1 à 20
R10 est OH, NH₂, N-R11R12,
R11 et R12, identiques ou différents, sont H, un alkyle en C₁-C₁₀ linéaire ou ramifié, saturé ou insaturé,
leurs mélanges d'énantiomères et/ou de diastéréomères, leurs sels pharmaceutiquement acceptables, leurs solvates et leurs hydrates, pour son utilisation dans le traitement du cancer.

2. Composition pharmaceutique à utiliser selon la revendication 1 dans laquelle, dans le composé de formule (I), PH1 et PH2 sont des phényles, R1 R2 et R5 sont CH₃, R6 R7 sont H, X est S, Y est N.

3. Composition pharmaceutique à utiliser selon la revendication 2, dans laquelle le composé de formule (I) est N-(1,5-diméthyl-3-oxo-2-phényl-2,3-dihydro-1H-pyrazol-4-YL)-3-méthyl-1-phényl-1H-thiéno[2,3-c]pyrazole-5-carboxamide. (C24H21N5O2S).

4. Composition pharmaceutique à utiliser selon la revendication 1, dans laquelle la composition pharmaceutique comprend en outre un médicament chimiothérapeutique.

5. Composition pharmaceutique à utiliser selon la revendication 4, dans laquelle le médicament chimiothérapeutique est choisi dans le groupe constitué par la doxorubicine, l'étoposide et le cisplatine.

6. Composition pharmaceutique à utiliser selon la revendication 1, dans laquelle le cancer est un cancer du sein, un cancer du poumon.
